Europäisches Patentamt

European Patent Office

Office européen des brevets

(19)

(11) Publication number: **0 121 399**
**A2**

# (12) EUROPEAN PATENT APPLICATION

(21) Application number: **84302074.4**

(22) Date of filing: **27.03.84**

(51) Int. Cl.³: **C 12 N 15/00**
**C 12 N 5/00**
**//C12P21/00**

(30) Priority: **30.03.83 US 480293**
**05.04.83 US 482240**

(43) Date of publication of application:
**10.10.84 Bulletin 84/41**

(84) Designated Contracting States:
**CH DE FR GB IT LI**

(71) Applicant: **BIO-RESPONSE INC.**
**550 Ridgefield Road**
**Wilton Connecticut 06897(US)**

(72) Inventor: **Rose, Sam**
**2701 Claremont Blvd.**
**Berkeley California 94705(US)**

(74) Representative: **Marsh, Roy David et al,**
**Brewer & Son 5-9, Quality Court Chancery Lane**
**London WC2A 1HT(GB)**

(54) A method for producing hybrids between myeloma cells and normal cells.

(57) A process for preparing a living, isolatable hybrid cell of a continuous dividing (e.g. malignant) cell and a normal cell not capable of continuous division, wherein a population of the malignant cells is mixed with a population of normal cells under conditions which result in the formation of one or more hybrids between a single malignant cell and a single normal cell, and wherein the population of the malignant cells, before mixed with the population of normal cells, is chemically treated so as to render the malignant cells incapable of living in a particular medium unless hybridized to normal cells.

EP 0 121 399 A2

B&S No. C84/25

# A METHOD FOR PRODUCING HYBRIDS BETWEEN
## MYELOMA CELLS AND NORMAL CELLS

The present invention relates to the production of living, isolatable hybrids between two living cells, preferably between malignant cells and normal cells, and, more particularly, to a process for producing monoclonal antibodies using the technique of hybridization.

In recent years, the art has developed the technique known as hybridization wherein a so-called hybrid cell is formed from two similar or dissimilar cell parents such that the hybrid cell exhibits desired characteristics of each of the parent cells. Typically, one of the chosen cell parents is a cell capable of continuous division, such as a malignant (e.g., myeloma) cell, the intent being that the hybrid cell itself will exhibit the desirable characteristic of continuous division, thereby resulting in the growth of a homogenous hybrid cell population (called a "hybridoma").

The other parent of the hybrid cell is a so-called "normal" cell which exhibits some desirable characteristic, but which is incapable of continuous division. The desirable characteristic of the normal cell typically is the production of a product, such as an antibody. The hybrid between a cell capable of continuous division and this normal, antibody-producing cell ideally exhibits the antibody production capability of its normal parent and the continuous division characteristic of its other parent, thereby constituting a cell which has the potential for producing large amounts of a desired antibody. For ease of reference in the following discussion, the cell parent capable of continuous division is taken for illustrative purposes to be a myeloma cell.

Hybridization of a myeloma cell and an antibody-producing cell is used in the preparation of so-called "monoclonal" antibodies and, indeed, the desire to prepare such antibodies in large part was the impetus behind development of the hybridization technique. A hybrid of a myeloma cell and an antibody-producing cell will produce only a single (monoclonal) product since the hybrid is made up of only a single antibody-producing cell and this single antibody-producing cell is itself capable of producing only a single type of product. Thus, when a population of myeloma cells is mixed under hybridization conditions with a population of antibody-producing cells (producing in toto many different products or antibodies), hybrids will form from a myeloma cell and a single antibody-producing cell. Each hybrid can then be grown up to a degree sufficient to produce a quantity of its particular product, and this product can then be tested to determine whether it is, e.g., an antibody to a particular antigen.

The efficacy of the hybridization technique in forming usable hybrids (such as those producing antibody) depends to a large degree on the ability to isolate such hybrids from other cells in the mixed populations of cells. Thus, for example, when a population of myeloma cells is mixed, under hybridization conditions, with a population of normal cells (producing antibody), the resultant population may contain the following cells:

    (a)   hybrid cells between a myeloma cell and a normal cell;

    (b)   hybrid cells between two myeloma cells;

    (c)   hybrid cells between two normal cells;

    (d)   unhybridized normal cells; and

    (e)   unhybridized myeloma cells.

Only the cells of category (a) are desired. The cells of categories (a), (b) and (e) can be "isolated" from those of categories (c) and (d) because, with time, the normal cells will eventually die since they are incapable of continuous division. However, the isolation of the cells of category (a) from those of categories (b) and (e) is more difficult since all such cells are capable of continuous division. To accomplish the isolation, the art resorts to the use of so-called "mutant" myeloma cells as the myeloma cell population in the hybridization process. The mutated myelomas possess a genetic deficiency in a material required for life in a particular medium. Mixing and hybridization of these cells to normal cells is then conducted in the above-noted medium (or, alternatively, after mixing and hybridization, the resultant cells are suspended in the above-noted medium). The unhybridized mutant myeloma cells and hybrids of two mutant myeloma cells eventually die in this medium while the desired hybrids of mutant myeloma cells and normal cells survive because the genetic deficiency of the mutant myeloma is provided by the normal cell component of the hybrid cell.

A specific example of this isolation method is the use of mutant myeloma cells deficient in hypoxanthine phosphoribosyl-transferase (HPRT). The growth of such cells is inhibited by selective hypoxanthine-aminopterin-thymidine (HAT) medium and, thus, suspension of all cells in this medium will result in the selective growth only of those mutant myeloma cells which have hybridized to a normal cell.

While the foregoing method produces effective isolation of hybrids between myeloma cells and normal cells, the need in such method for mutant myeloma cells exhibiting some genetic deficiency places restrictions on the hybridization process *per se*. On the one hand, the finding and isolation of such mutant myelomas can be an extremely laborious task involving time and expense which significantly detract from the economics of hybridization as a means for, e.g., producing monoclonal antibodies. On the other hand, the known mutant myeloma cell lines are relatively few and this, therefore, places undesirable strictures on the types of cells, media, etc. which can be employed in the hybridization process.

It is an object of the present invention to provide a process for the production or formation of a hybrid between a cell capable of continuous division and a normal cell, which process does not depend upon the preliminary production or availability of particular mutant cells.

Another object of the present invention is to provide a process, as described above, for the production of monoclonal antibodies.

These and other objects are achieved through the provision of a process for forming hybrids between a cell capable of continuous division and a normal cell, wherein the continuous dividing cells employed in the process, rather than being those resulting from a genetic mutation, have been chemically treated to inactivate a component of such cells required for life in a particular medium.

A particular example of the process according to the present invention involves the treatment of myeloma cells with an agent (e.g., methotrexate or aminopterin) which inactivates the dihydrofolic acid reductase of the myeloma cells. Dihydrofolic acid reductase is an enzyme, present in all living cells, which converts dihydrofolic acid to folinic acid, the latter being a material required by all mammalian cells for life. Accordingly, myeloma cells in which dihydrofolic acid reductase has been inactivated (through binding thereto of methotrexate or aminopterin) cannot live in a medium containing dihydrofolic acid because these treated myeloma cells are incapable of converting the dihydrofolic acid to the required folinic acid. However, treated myeloma cells which have hybridized to a normal cell will survive in such a medium because the normal cell component of the hybrid provides to the hybrid the dihydrofolic acid reductase needed to produce the necessary folinic acid.

In the following discussion, there first is presented a somewhat generalized description of the hybridization process per se utilizing cells chemically treated as above-noted. Thereafter, details with respect to the chemical treatment are provided.

In the hybridization process, there is provided a population of "continuous dividing" cells and a population of "normal" cells, the latter possessing some desirable characteristic sought to be carried through to the hybrid cell where, because of the continuous division characteristic of the continuous dividing parent of the hybrid, the desirable characteristic of the normal cell potentially can be perpetuated.

The continuous dividing cell for use in the present invention is any cell which possesses the capability of continous growth in all or particular media or environments and whose ability for such continuous growth can be imparted to a hybrid made therefrom. Typically, the continuous dividing cell is a malignant cell such as a myeloma cell. Alternatively, however, the continuous dividing cell may be a non- malignant cell which, nevertheless, exhibits continuous growth in the presence of particular compounds or media, such as T-cells in the presence of T-cell growth factor. In this latter case, hybrids between a normal cell and a T-cell will exhibit the desired continuous growth when the hybrid cell is in the presence of T-cell growth factor.

The known prior art processes require that the continuous dividing cell also be a mutant in order to enable the isolation from all other cells of the hybrids of such cells with normal cells. The process of the present invention is not so restricted, since the inability of an unhybridized continuous dividing cell to survive in a particular environment is achieved by overt chemical pre-treatment of the continuous dividing cell.

Again, for ease of discussion, the continuous dividing cell in the following description is taken for illustration to be a malignant myeloma cell.

Also for purposes of illustration, the population of normal cells is taken to be a population of cells, at least some of which are producing a specific desired antibody sought to be isolated and collected. The remaining cells of the population of normal cells may either be producing no products at all or may be producing products (e.g., antibodies) other than the specific antibody desired.

The population of normal cells as above-defined may be obtained by a number of methods known in the art, such as:

(a) immunizing an organism with the antigen specific to the desired antibody and isolating the cells from the organism (e.g., by collecting the lymphocytes from the immunized organism);

(b) isolating the cells produced by an organism in response to a disease state which exhibits the antigen specific to the desired antibody. For example, a tumor-bearing or tumor-cured organism will have antibody producing cells making antibody against the tumor antigen;

(c) stimulating normal cells of an organism in vitro with the antigen specific to the desired antibody; and

(d) treating normal cells of an organism in vitro with a chemical capable of stimulating the normal cells to produce the full complement of antibodies which the cells of that organism are genetically able to produce, including the desired antibody. Examples of such chemicals are lipopolysaccharides and various plant lectins.

The population of normal cells and the population of treated myeloma cells are then, according to one form of the hybridization process, mixed together in a suitable vessel(s) in the presence of a "fusing" agent, such as polyethyleneglycol (PEG) or inactivated Sendai virus. By virtue of the fusing agent, the cells of the mixed populations are randomly caused to stick or attach to one another to form a series of fused cells or doublets. A proportion of these doublets form hybrids. After sufficient time has been permitted for hybridizations to occur, all cells (inter alia, hybrids of myeloma and normal cells, hybrids of two myeloma cells or two normal cells and unhybridized normal or myeloma cells) are suspended in a medium in which, because of the chemical pre-treatment of the myeloma cell population, myeloma cells not hybridized to a normal cell cannot survive. At the same time, normal cells not hybridized to myeloma cells will die because they cannot divide continuously. The remaining living and dividing cells (i.e., myeloma/normal cell hybrids) can then be grown up from single hybrids to a substantial number and the fluids in which these cells are growing are tested to see if a particular hybridoma is one making the specific antibody desired.

In another form of the hybridization process *per se*, the reliance upon fusing agents to achieve the initial cell attachments is avoided. This process is described in detail in my co-pending U.S. patent application Serial No. 90,130, filed November 1, 1979 and entitled, "A Method For The Production Of Monoclonal Antibodies." A European Patent corresponding to U.S. application Serial No. 90,130 was granted as Patent No. 0028902 on May 18, 1983, and is incorporated herein by reference. Briefly, the process was developed in recognition of the fact that even the successful isolation of all hybrids between myeloma cells and normal cells (such as by the use of mutant myeloma cells or, as in the present invention, chemically pre-treated myeloma cells) still leaves the need to isolate from these hybrids only the hybrids making the specific antibody desired. This is because the fusing agents used in achieving initial cell attachments are random and non-specific; hence, a number of isolatable hybrids of myeloma and normal cells will result, but only a very small proportion of these hybrids will be between a myeloma cell and a normal cell making the specific antibody desired. Even as to the latter, the production of specific antibody by the normal cell may not for some reason have been carried through to the hybrid. In the known hybridization method employing fusing agents, the final "isolation" of only those hybrids making the specific antibody desired can only be accomplished by growing up each and every surviving hybrid and testing the fluid therefrom.

In the process of application Serial No. 90,130, the myeloma cells employed in the hybridization process are provided, either attached to or as part of their surface, with the antigen specific to the specific antibody desired. In the absence of fusing agents, therefore, a mixture of such myeloma cells and normal cells will result in attachments (and eventual hybridization) only between myeloma cells and those cells of the normal cell population which are producing the specific (desired) antibody. These latter cells have an antibody-like receptor on their surface which will attach to the antigenic receptor provided on the myeloma cells. As a result of this technique, the number of desired hybrids (i.e., those actually making the specific antibody desired), relative to all hybrids produced, is very large and such desired hybrids are, therefore, much more easily isolated.

Regardless of the hybridization technique employed, the present invention provides for the use of continuous dividing (e.g., myeloma) cells which have been chemically pre-treated to render such cells incapable of living in some predetermined medium. By virtue of this treatment, suspension in such a medium of the cells from the hybridization process will result in the selective death of all cells except the hybrid cells between continuous dividing cells and normal cells.

As earlier-noted, distinct advantages of the process of the present invention are avoidance of the need for preparing or obtaining genetically mutated continuous dividing cells and "opening up" the hybridization process to a much wider variety of continuous dividing cells, media and other conditions. By way of example, the mutant myelomas capable of use in known hybridization processes are few, both because of the difficulty of isolating such mutants and the need to insure that the particular mutation can be manifested in the form of an inability of the mutant to live in a medium in which all the cells from the hybridization process can be suspended. In the process of the present invention, any number of pre-treatments can be practiced on the continuous dividing cells which alter the cells in some manner capable of being manifested in the inability of the treated cells to survive a particular medium (unless hybridized to a normal cell).

Particularly preferred at present is the chemical treatment of continuous dividing cells to irreversibly inactivate dihydrofolic acid reductase.

According to the present invention, therefore, the entire continuous dividing cell population is chemically treated and then employed in any of the known hybridization techniques. Choice of the appropriate medium for thereafter suspending the cells results in the isolation of hybrids between normal cells and continuous dividing cells, from which, if required, particular desired hybrids, (i.e., those making a particular product) can be further isolated.

0121399

- 11 -

CLAIMS

1.        A process for producing a living, isolatable hybrid between a continuous dividing cell and a normal cell incapable of continuous division, wherein a population of said continuous dividing cells is mixed with a population of said normal cells under conditions which result in the production of one or more hybrid cells between a single, continuous dividing cell and a single normal cell, and wherein all the living cells remaining after said mixing and hybrid production are exposed to a predetermined medium in which, by virtue of the particular continuous dividing cells employed, only hybrids between a continuous dividing cell and a normal cell survive, characterised in that said population of continuous dividing cells consists of continuous dividing cells which cannot survive in the predetermined medium by virtue of a component of such cells, necessary for life in said medium, having been chemically inactivated prior to the mixing of the population of continuous dividing cells and the population of normal cells for the purpose of hybridization.

2.        A process according to claim 1 characterised in that the continuous dividing cells have been chemically inactivated by a plurality of chemical pre-treatments.

3.        A process according to claim 1 or 2, characterised in that the continuous dividing cells are malignant myeloma cells.

4.      A process according to claim 1 or 2, characterised in that the continuous dividing cells are non-malignant cells which exhibit continuous growth in the presence of a predetermined compound.

5.      A process according to claim 4, characterised in that the non-malignant cell is a T-cell and in which the predetermined compound is T-cell growth factor.

6.      A process according to claim 3, characterised in that the predetermined medium contains dihydrofolic acid but no folinic acid and wherein said myeloma cells have had their dihydrofolic acid reductase chemically inactivated.

7.      A process according to claim 6, characterised in that the chemical inactivation of said dihydrofolic acid reductase is achieved by suspending population of myeloma cells in an agent which inactivates the dihydrofolic acid reductase prior to mixing said population with the population of normal cells for the purpose of hybridization.

8.      A process according to claim 7, characterised in that the agent is a substance selected from the group consisting of methotrexate and aminopterin.